# EUROPEAN PATENT SPECIFICATION

(11) **EP 1 813 209 B1**
(45) Date of publication and mention of the grant of the patent: **04.08.2010**
(21) Application number: 07250384.0
(22) Date of filing: 30.01.2007
(51) Int. Cl.: A61B 17/072

(54) **Disposable staple cartridge having an anvil with tissue locator for use with a surgical cutting and fastening instrument and modular and effector system therefor**
Wegwerfklammerkassette mit Block zur Gewebelokalisierung zur Verwendung mit einem chirurgischen Schneide- und Befestigungsinstrument und ein modulares Effektorsystem dafür
Cartouche à agrafe jetable ayant un incus avec un localisateur de tissu à utiliser avec une taille chirurgicale et un instrument d'attache, et un système modulaire et effecteur correspondant

(30) Priority: 31.01.2006 US 343546
(43) Date of publication of application: 01.08.2007
(73) Proprietor: ETHICON ENDO-SURGERY, INC., Cincinnati, OH 45242-2839 (US)
(72) Inventor: Shelton, IV, Frederick E., New Vienna, Ohio (US); Cropper, Michael S., Edgewood KY (US); Crisp, Ryan S., Lewis Center OH (US); Float, Jamison J., Westerville OH (US); Broehl, Joshua M., Worthington OH (US); Timperman, Eugene L., Cincinnati OH (US)
(74) Representative: Tunstall, Christopher Stephen

(56) References cited:
- EP-A- 0 033 548
- EP-A- 0 484 677
- EP-A- 0 760 230
- EP-A- 0 829 235
- US-A- 4 520 817
- US-A- 4 715 520
- US-A1- 2001 002 029

## Description

### BACKGROUND

The present invention generally concerns surgical instruments and, more particularly, surgical cutting and fastening instruments.

Endoscopic surgical instruments are often preferred over traditional open surgical devices since a smaller incision tends to reduce the post-operative recovery time and complications. Generally, these endoscopic surgical instruments include an "end effector", a handle assembly and an elongated shaft that extends between the end effector and the handle assembly. The end effector is the portion of the instrument configured to engage the tissue in various ways to achieve a desired diagnostic or therapeutic effect (e.g., endocutter, grasper, cutter, staplers, clip applier, access device, drug/gene therapy delivery device, and energy device using ultrasound, RF, laser, etc.).

The end effector and the shaft portion are sized to be inserted through a trocar placed into the patient. The elongated shaft portion enables the end effector to be inserted to a desired depth and also facilitates some rotation of the end effector to position it within the patient. With judicious placement of the trocar and use of graspers, for instance, through another trocar, often this amount of positioning is sufficient. Surgical stapling and severing instruments, such as those described in U.S. Pat. No. 5,465,895, are examples of an endoscopic surgical instrument that successfully positions an end effector by insertion and rotation.

Two primary design approaches have been used in the past by various medical component manufacturers to reduce the overall cost of such endoscopic surgical instruments. While both attempts have been successful at addressing at least some of the user's needs, neither attempt has fully addressed all of those needs.

For example, the first design approach concerned the use of a disposable end effector for each firing. Such end effectors were fabricated from relatively weak components that can only be fired once before a new end effector is required since the end effector components are markedly deformed after each firing. This type of end effector employed an "I-beam" feature on the knife to prevent the anvil from deflecting away from the cartridge during firing. Since the I-beam is riding on a relatively weak anvil component, the I-beam deforms the portion of the anvil located directly underneath the I-beam feature. This combination of factors allows staple form to be improved while decreasing the forces required to actuate the device because the I-beam is only pulling the anvil material down to the correct position relative to the cartridge where its needed to form a staple at any given time.

While this approach improves staple form and lowers the force required to cut the tissue and deploy the staples, it has a number of disadvantages. First, having the surgeon dispose of an entire anvil, channel and cartridge after each firing adds a great deal of manufacturing expense. Second, the device often doesn't securely clamp the targeted tissue due to a combination of the relatively weak end effector components and the manner in which the anvil is actually clamped onto the tissue. The third issue is that the relatively weak end effector components can only reliably be used to fire on moderate tissue thicknesses and the components will not function on very thick tissues.

The second primary design approach taken in the past is to make much stronger end effector components that can be used for multiple firings. In this approach, only the cartridge assembly is replaced after each firing. Because the end effector components have to resist multiple firing loads, they are purposefully designed such that they do not deform after each firing. This design concept employs a different set of features on the knife to keep the anvil positioned relative to the cartridge during deployment of the staples to improve staple form. The combination of the stiffer end effector components and a different clamping mechanism allows this type of device to reliably exert high clamping loads so the surgeon can easily manipulate the desired tissue. The stiffer components also allow the end effector to be reliably used on relatively thick tissue samples.

One disadvantage of this approach, however, is that the features on the knife that prevent the anvil from deflecting away from the cartridge are now required to pull a very stiff anvil component down towards the cartridge. The knife is therefore attempting to pull an entire beam down towards the cartridge that is as long as the entire staple line instead of just pulling the anvil down in a localized region as on the previous device. This approach, therefore, generally must generate higher deployment forces due to the increased anvil drag loads on the knife.

Consequently there is a need for an end effector arrangement for a surgical cutting and fastening instrument that addresses the above-mentioned concerns by maintaining the same staple form quality, clamping performance and the ability to repeatedly deploy staples in thick tissue while lowering the amount of force required to deploy the staples and knife.

There is still another need for an end effector system that can be used with staple cartridges that are of identical length but have different staple line lengths.

EP-A-484677 discloses a disposable staple cartridge/anvil unit as in the preamble to appended claim 1.

### SUMMARY

In one general aspect, the present invention is directed to a disposable staple cartridge for a surgical cutting and fastening instrument as in appended claim 1.

The disposable anvil may be fabricated from thin deflectable plate material.

A plurality of staple forming pockets corresponding to said plurality of staples in said cartridge body may be formed in said bottom surface of said disposable anvil.

A proximal end of said disposable anvil may be attached to a proximal end of said cartridge body by at least one spring.

The at least one tissue stop may comprise a first tissue stop protruding downwardly from one lateral side of said disposable anvil and a second tissue stop protruding downwardly from a second lateral side of said disposable anvil.

The disposable anvil may have a longitudinally extending slot portion therein to enable a portion of a knife assembly of the surgical cutting and fastening instrument to protrude therethrough such that lateral extending guide tabs on the knife assembly serve to deflect the disposable anvil toward said top surface of said cartridge body as the knife assembly is driven through said cartridge body and said longitudinally extending slot in said disposable anvil.

The disposable anvil may be fabricated from sheet metal material.

The disposable staple cartridge may further comprise a plurality of staple drivers operably supported in said cartridge body, each said staple driver supporting at least one staple thereon and being movably supported within said cartridge body such that when contacted by a driven wedge cam of the surgical cutting and fastening instrument, each said staple driver drives the staples supported thereon into forming contact with said disposable anvil.

### DRAWINGS

Various embodiments of the present invention are described herein by way of example in conjunction with the following figures, wherein like numerals may be used to describe like parts and wherein:

Figure 1 is a perspective view of a surgical cutting and fastening instrument that can employ various end effector embodiments and staple cartridge embodiments of the present invention;

Figure 2 is an exploded assembly view of an end effector embodiment of the present invention;

Figure 3 is a cross sectional view of an end effector embodiment of the present invention supporting a staple cartridge embodiment of the present invention therein with some of the components thereof omitted for clarity;

Figure 4 is a partial top view of a staple cartridge embodiment of the present invention;

Figure 5 is a partial cross-sectional view of a staple cartridge embodiment and end effector of the present invention illustrating the firing of staples into tissue clamped in the end effector;

Figure 6 is a bottom perspective view of an end effector embodiment and staple cartridge embodiment of the present invention with the elongate channel removed therefrom;

Figure 7 is a partial perspective view of an end effector embodiment and a staple cartridge embodiment of the present invention;

Figure 8 is a perspective view of a staple cartridge embodiment of the present invention installed in an end effector embodiment of the present invention in an open position;

Figure 9 is a cross-sectional view of the end effector and staple cartridge depicted in Figure 8;

Figure 10 is a perspective view of the end effector and staple cartridge depicted in Figures 8 and 9;

Figure 11 is a cross-sectional view of the staple cartridge and end effector of various embodiments of the present invention in an open position prior to clamping a piece of tissue therein;

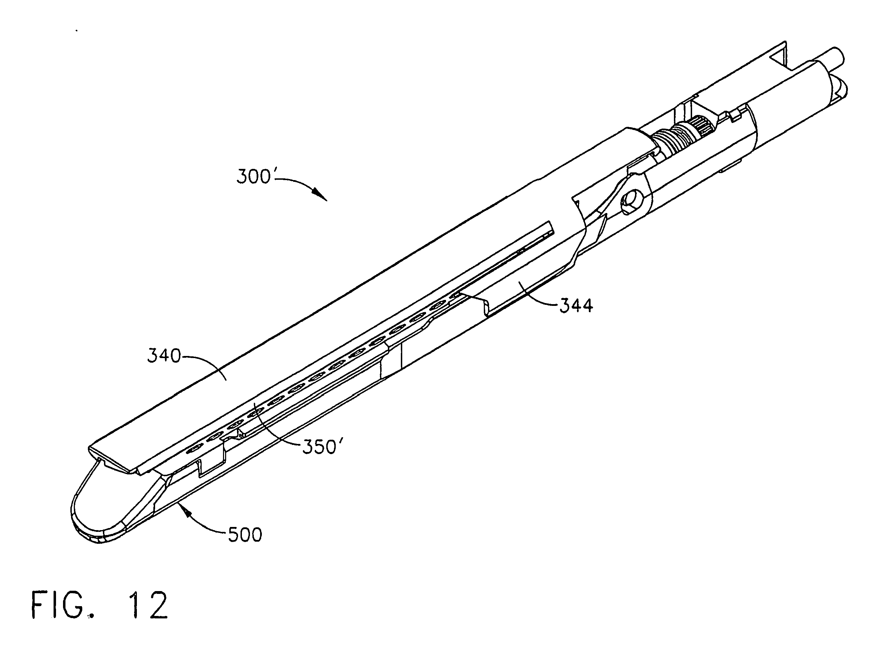

Figure 12 is a perspective view of an end effector and staple cartridge of another embodiment of the present invention in a closed position;

Figure 13 is another perspective view of the end effector and staple cartridge embodiment of Figure 12 illustrating the position of the bottom anvil plate thereof prior to removal from the end effector;

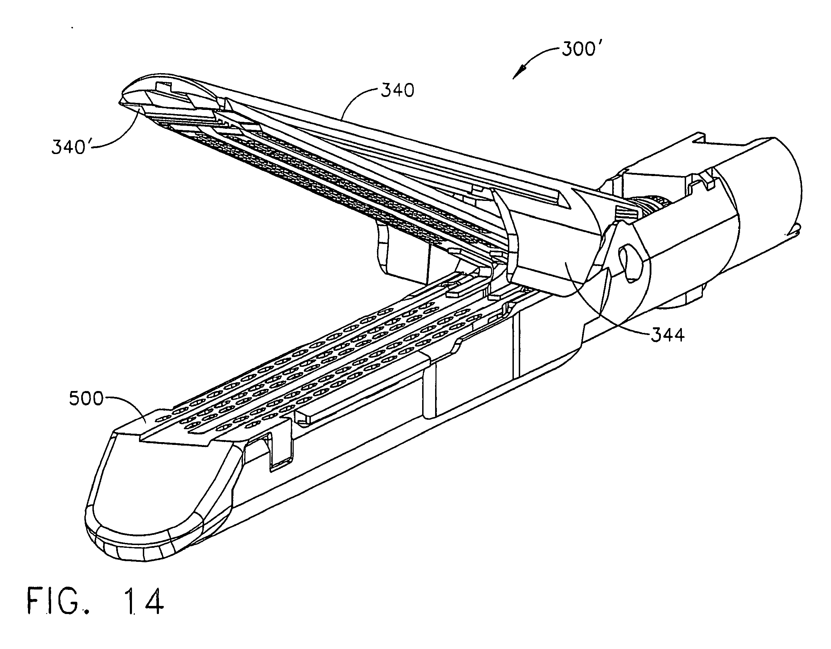

Figure 14 is a perspective view of the end effector and staple cartridge of Figures 12 and 13 in the open position;

Figure 15 is a cross-sectional view of the end effector and staple cartridge depicted in Figure 14;

Figure 16 is a partial enlarged cross-sectional view of an end effector and staple cartridge of various embodiments of the present invention in an open position;

Figure 17 is a cross-sectional view of an end effector and cartridge assembly of various embodiments of the present invention in a closed position;

Figure 18 is a partial enlarged cross-sectional view of the end effector and staple cartridge of Figure 17;

Figure 19 is a perspective view of a distal drive shaft portion of various embodiments of the present invention;

Figure 20 is a cross-sectional view of the distal drive shaft portion of Figure 19;

Figure 21 is a perspective view of a tapered clutch member of various embodiments of the present invention;

Figure 22 is a cross-sectional view of the tapered clutch member of Figure 21;

Figure 23 is a perspective view of a clutch plate of various embodiments of the present invention;

Figure 24 is a cross-sectional view of the clutch plate of Figure 23;

Figure 25 is a perspective view of a closure nut of various embodiments of the present invention;

Figure 26 is a cross-sectional view of the closure nut of Figure 25;

Figure 27 is a cross-sectional view of the distal drive shaft portion and closure nut with the closure nut in an open position; and

Figure 28 is another cross-sectional view of the distal drive shaft portion and closure nut with the closure nut in the closed position.

### DETAILED DESCRIPTION

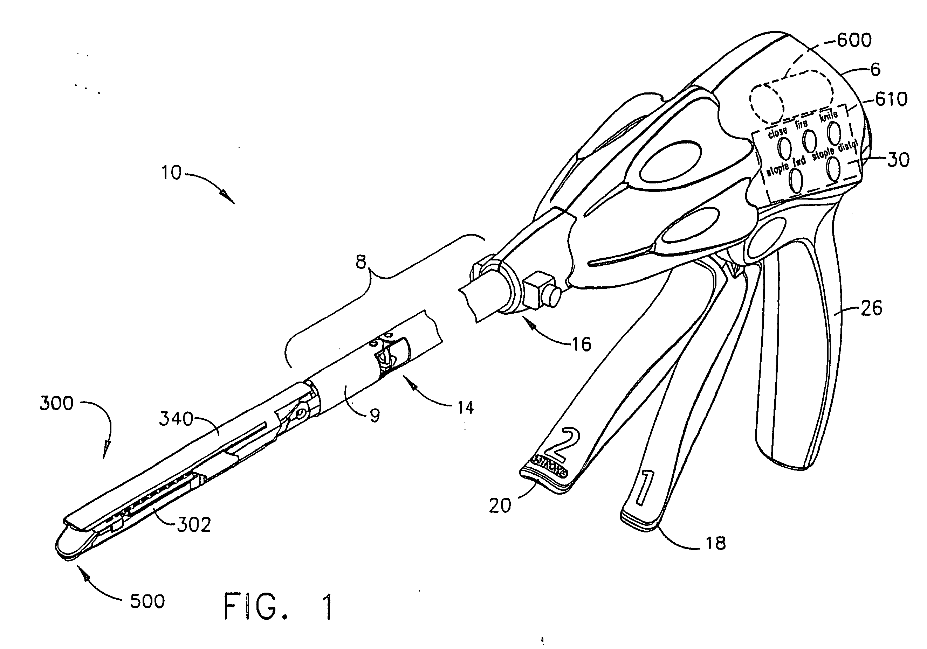

Figure 1 depicts a surgical cutting and fastening instrument 10 that is capable of practicing various unique benefits of the end effector arrangements and/or staple cartridge arrangements of the present invention. The surgical instrument 10 depicted in Figure 1 comprises a handle 6, a shaft assembly 8, and an articulating end effector 300 pivotally connected to the shaft assembly 8 at an articulation pivot 14. In various embodiments, the control handle houses a drive motor 600 and control system generally represented as 610 therein for controlling the opening and closing of the end effector 300 and the cutting and stapling of the tissue clamped therein. An articulation control 16 may be provided adjacent to the handle 6 to effect rotation of the end effector 300 about the articulation pivot 14. The handle 6 of the instrument 10 may include a closure trigger 18 and a firing trigger 20 for actuating the end effector 300. The end effector 300 is shown separated from the handle 6 by a preferably elongate shaft 8. In one embodiment, a clinician or operator of the instrument 10 may articulate the end effector 300 relative to the shaft 8 by utilizing the articulation control 16, as described in more detail in pending United States Patent Application US-A1-2007 158 385. Other articulation arrangements could also be employed.

As will be discussed in further detail below, various end effector embodiments include a pivotally translatable anvil assembly, which is maintained at a spacing that assures effective stapling and severing of tissue clamped in the end effector 300. In various exemplary embodiments, the handle 6 may include a pistol grip 26 towards which a closure trigger 18 is pivotally drawn by the clinician to cause clamping or closing of an a top anvil plate 340 toward cartridge 500 seated in an elongate channel 302 of the end effector 300 to thereby clamp tissue positioned between the top anvil plate 340 and the staple cartridge 500. A firing trigger 20 may be situated farther outboard of the closure trigger 18. In various embodiments, once the closure trigger 18 is locked in the closure position as further described below, the firing trigger 20 may rotate slightly toward the pistol grip 26 so that it can be reached by the operator using one hand. Then the operator may pivotally draw the firing trigger 20 toward the pistol grip 26 to cause the stapling and severing of clamped tissue in the end effector 300. Those of ordinary skill in the art will readily appreciate however, that other handle and drive system arrangements may be successfully employed in connection with various embodiments described herein and their equivalent structures without departing from the spirit and scope of the present invention.

It will be appreciated that the terms "proximal" and "distal" are used herein with reference to a clinician gripping the handle 6 of an instrument 10. Thus, the end effector 300 is distal with respect to the more proximal handle 6. It will be further appreciated that, for convenience and clarity, spatial terms such as "vertical" and "horizontal" are used herein with respect to the drawings. However, surgical instruments are used in many orientations and positions, and these terms are not intended to be limiting and absolute.

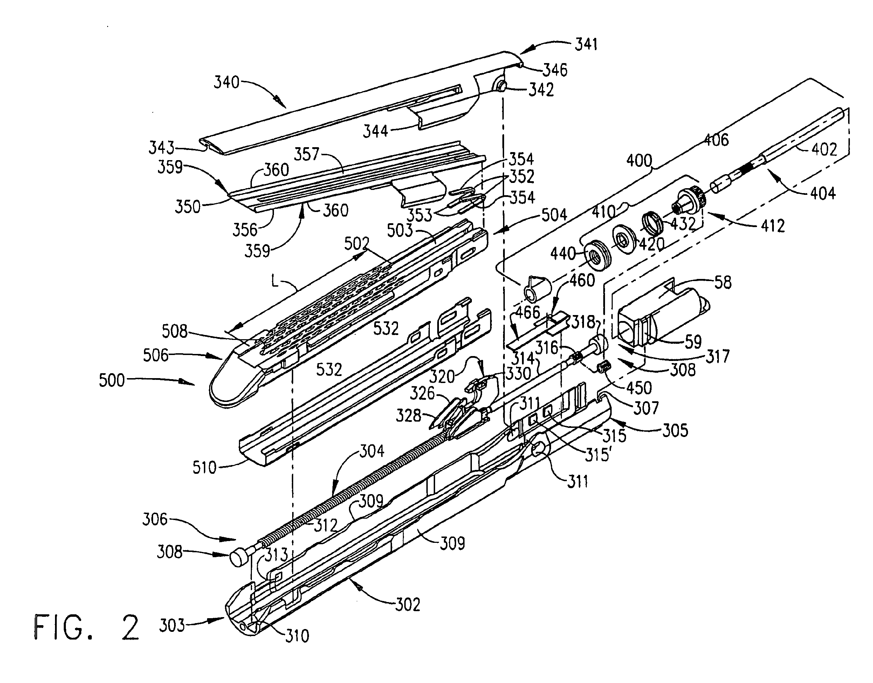

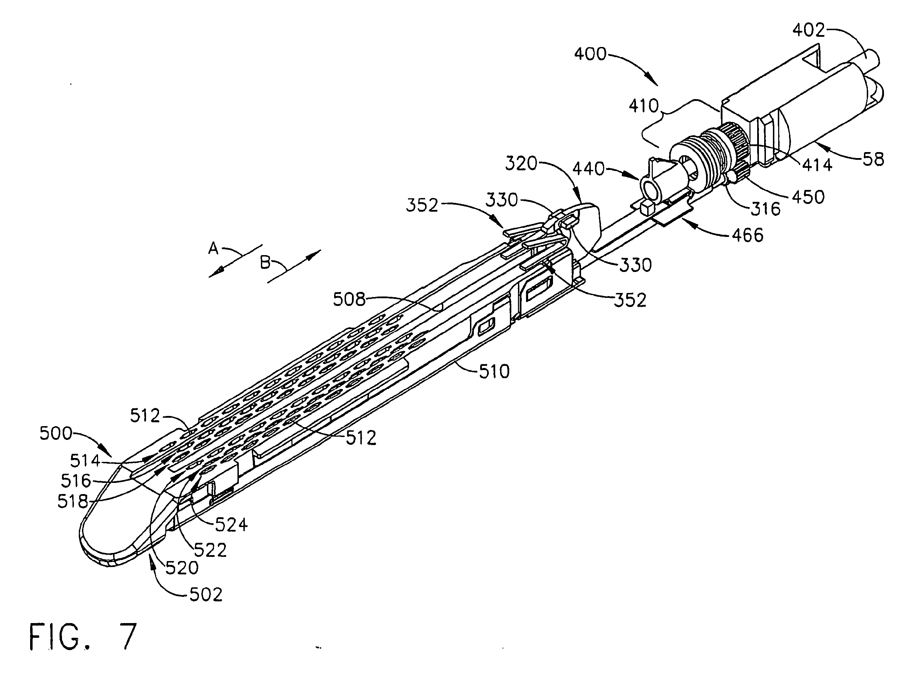

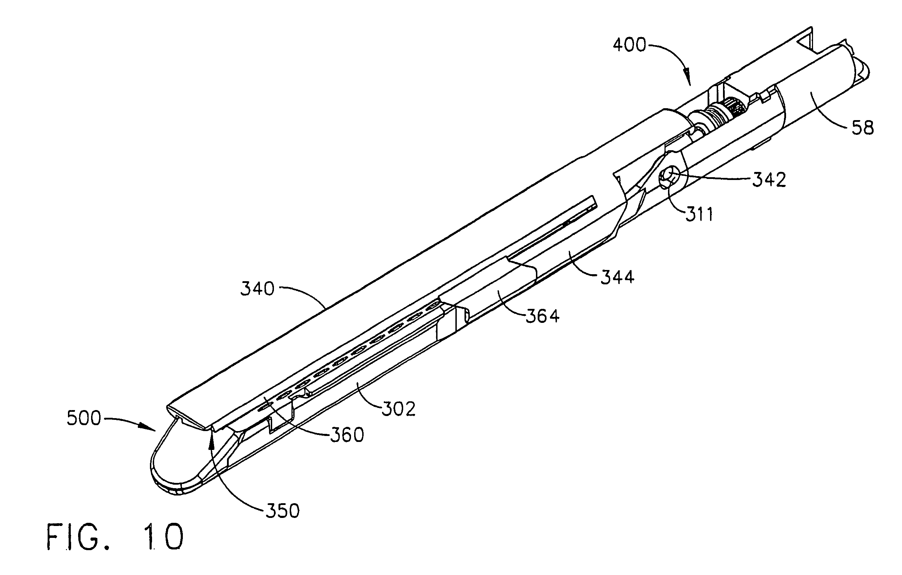

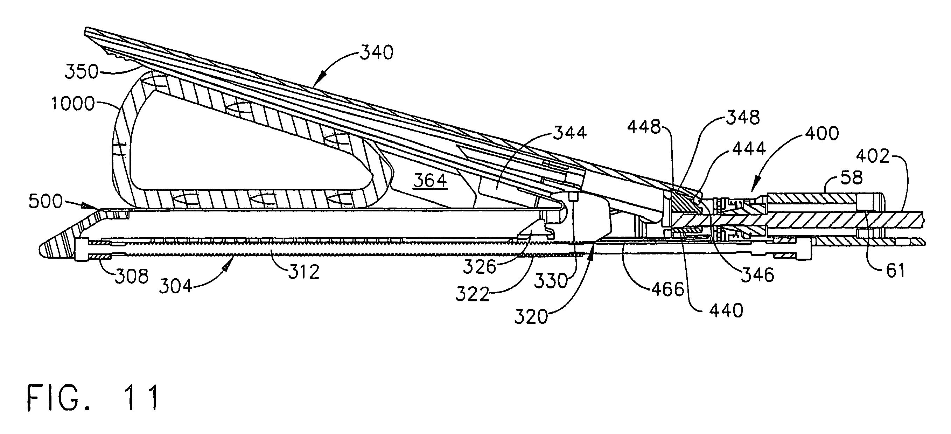

Figures 2-11 illustrate a unique and novel end effector 300 of various embodiments of the present invention. As the present Detailed Description proceeds, the reader will appreciate that the end effector 300 in Figures can accommodate different staple cartridges 500. For example, the end effector 300 may accommodate 45mm and 60mm disposable staple cartridges that have cartridge bodies that are of the same length. Such staple cartridges bodies and their operation are known in the art and thus will not be discussed in great detail herein. For example, U.S. Patent No. U.S. Pat. 6,978,921, entitled "Surgical Stapling Instrument Incorporating an E-beam Firing Mechanism" provides more details about the construction of such staple cartridges.

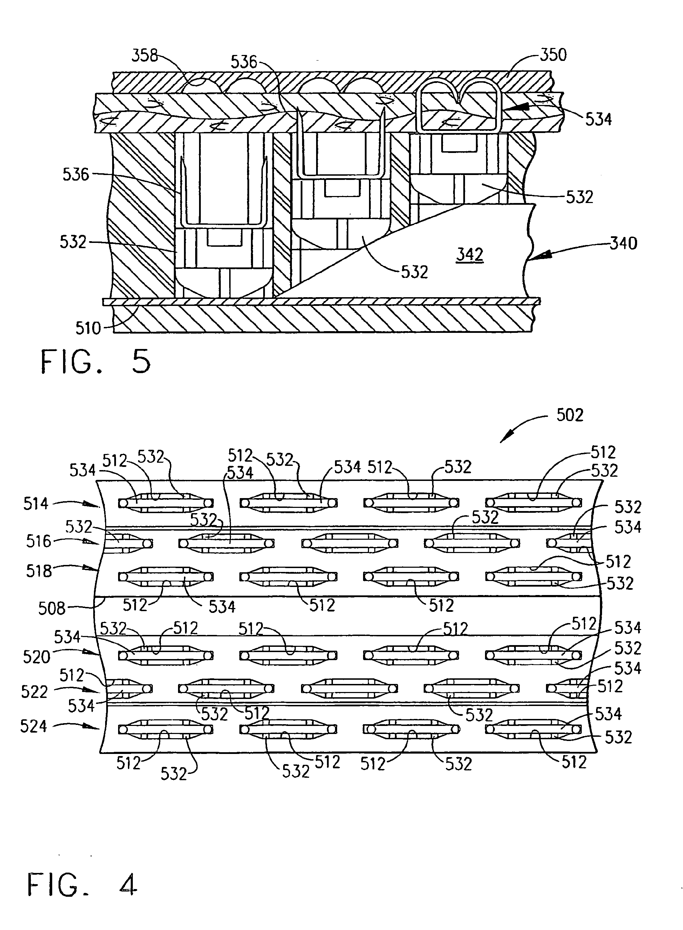

In general, various staple cartridges 500 include a cartridge body 502 that is divided by a central, elongated slot 508 which extends from the proximal end 504 of the cartridge body 502 towards its tapered outer tip 506. See Figure 2. The cartridge body 502 may be fabricated from a polymeric material and be attached to a metal cartridge pan 510. In various arrangements, the cartridge body 502 for a 45mm cartridge for example would have a length that is the same as the length "L" of a cartridge body 502 for a 60mm cartridge. A plurality of staple receiving pockets 512 are formed within the cartridge body 502 and are arranged in six laterally spaced longitudinal rows or "lines" of staples 514, 516, 518, 520, 522, 524. See Figure 5. Also in various embodiments, the length "L" of the lines of staples for a 45mm cartridge would be shorter than the length "L" of the lines in a 60 mm cartridge. The skilled artisan will readily appreciate that the 45mm and 60mm sized cartridges are used herein as examples only. Other cartridge sizes with different lines of staples may embrace the unique and novel aspects of various embodiments of the present invention.

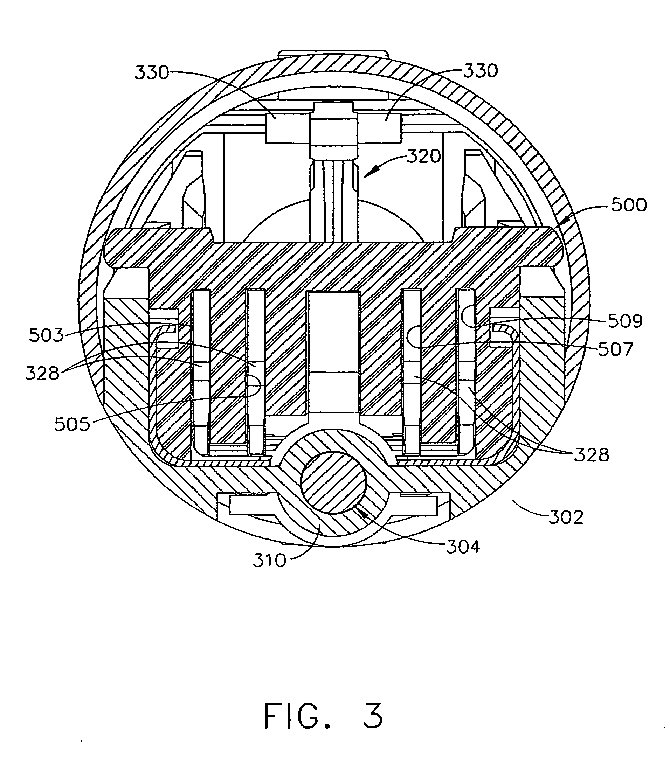

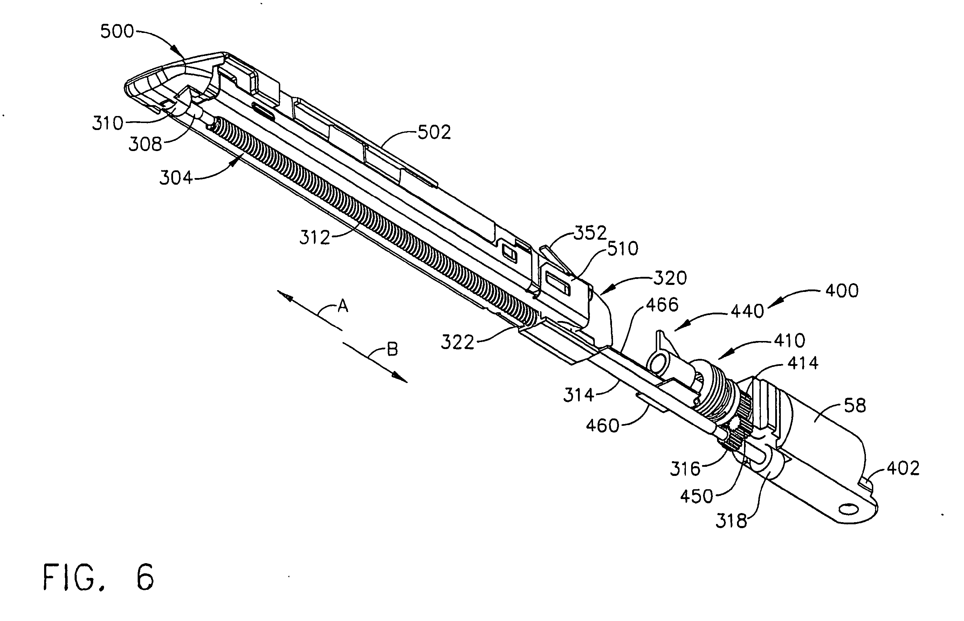

Positioned within the pockets 512 are staple-supporting drivers 532 which support staples 534 thereon. Depending upon the location (line) of staple-receiving pockets 512, the staple supporting drivers 532 may support one or two staples 530 thereon. The cartridge body 502 further includes four longitudinal slots 503, 505, 507, 509 extending from its proximal end 504 to its tapered outer tip 506 for receiving corresponding sled cams 328 formed on a wedge sled 326 in the end effector 300, the construction and operation of which will discussed in further detail below. See Figure 3. As the sled cams 342 are advanced through their respective slots 503, 505, 507, 509 in the cartridge body 502 from proximal end 504 to distal end 506, they contact the staple-supporting drivers 532 associated with those slots and force the staple-supporting drivers 532 and the staples 534 that they support upward out of the cartridge body 502. See Figure 6. As the ends of the legs 536 of the staple 534 contact the pockets 358 formed in the bottom anvil plate 350, they are folded over to close the staples 534.

As the present Detailed Description continues, the reader will also appreciate that various unique and novel aspects of various embodiments of the present invention enable many of the end effector components to fabricated from sheet metal to thereby reduce the overall costs of the end effector. Other end effector components may comprise machined parts.

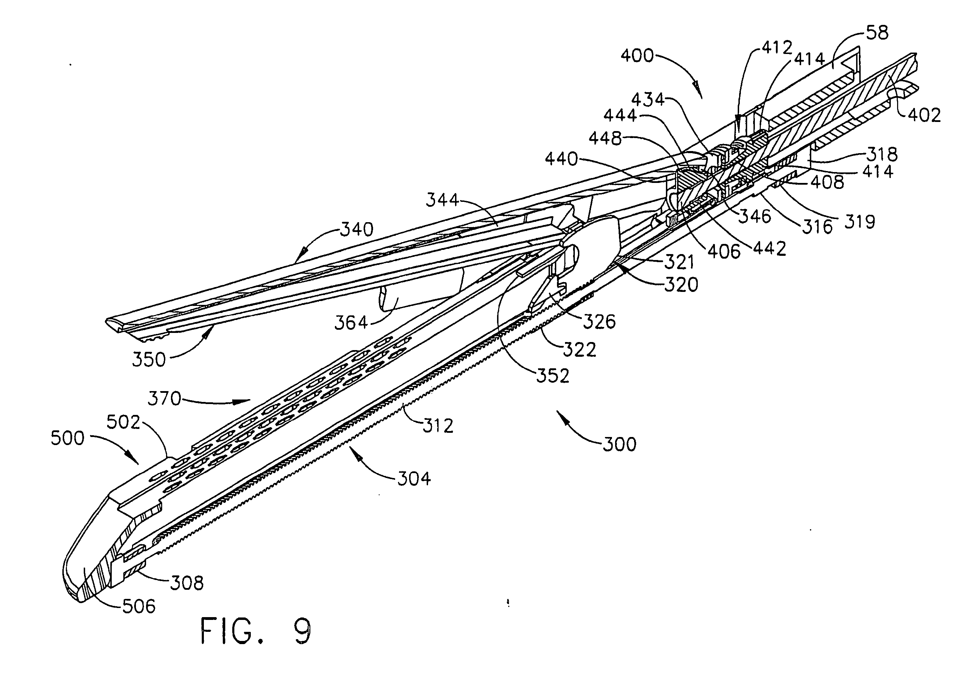

Various end effectors of the present invention include an elongate channel 302 that is sized to removably receive and support the cartridge body 502 of a disposable cartridge 500 therein. The reader will understand that in various embodiments, the elongate channel 302 is configured to support any one of the like sized staple cartridge bodies regardless of the length of the lines of staples supported therein. A knife screw 304 is rotatably supported in the elongate channel 302. The knife screw 304 has a distal end 306 that has a distal thrust bearing 308 attached thereto that is rotatably supported by a distal bearing housing 310 formed in the distal end 303 of the elongate channel 302. See Figure 2. The knife screw 304 has a central drive portion 312 with a helical thread formed thereon. The knife screw 304 further has a smooth extension portion 314 and a knife screw gear 316 formed thereon or otherwise attached thereto. A proximal thrust bearing 318 is formed or attached to the proximal end 317 of the knife screw 304. The proximal thrust bearing 318 is rotatably housed within a proximal bearing housing 319 supported in a distal spine tube segment 58. See Figure 9. The distal spine tube segment 58 has a pair of columns 59 formed on its distal end that are adapted to be received in vertical slots 307 formed in the proximal end 305 of the elongate channel 302. The columns 59 may be retained within the slots 307 in the elongate channel 302 by friction, adhesive, or by the distal end of the shaft tube 9. See Figure 1.

Various embodiments of the present invention further include a knife assembly 320 that has a knife/sled bearing 322 that is threaded onto the threaded portion 312 of the knife screw 304. The knife assembly 320 supports a vertically extending blade 324 and a wedge sled 326 that supports the four sled cams 328. The reader will understand that, as the knife screw 304 is rotated in a clockwise direction, the knife assembly 320 and the wedge sled 326 is advanced toward the distal end 303 (direction "A") of the elongate channel 302 and, when the knife screw 304 is rotated in a counterclockwise direction, the knife assembly 320 and wedge sled 326 is moved toward the proximal end 305 of the channel member 302 (direction "B"). As can be seen in Figure 9, in various embodiments, the knife assembly 320 has a flat bearing portion 322 thereon that is adapted to slide on the inside surface of the bottom of the elongate channel 302 and provide support to the knife assembly 320 and wedge sled 326 as they are advanced within the elongate channel 302. In addition, the knife assembly 320 has a pair of laterally extending deflector tabs 330 protruding therefrom, the purpose of which will be discussed below.

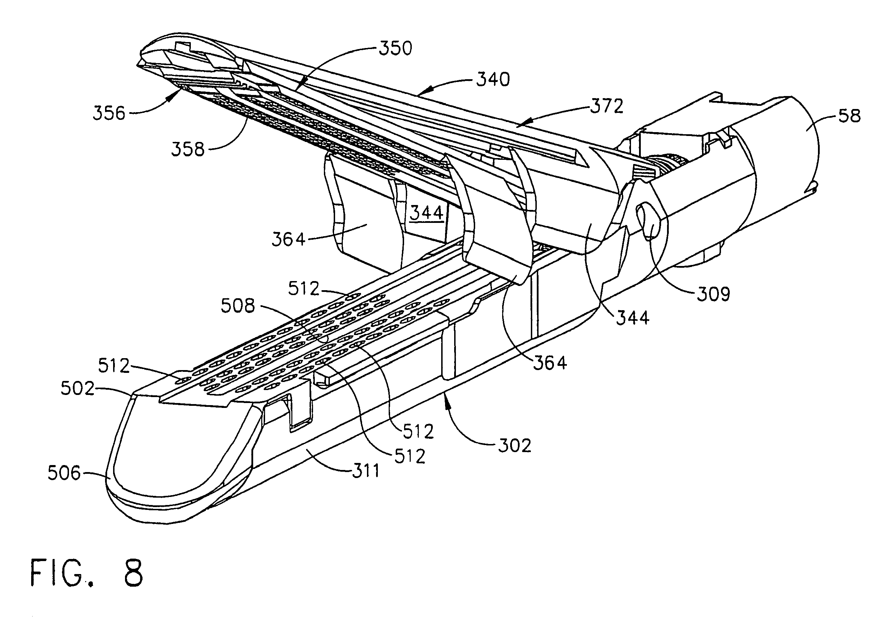

In various embodiments of the present invention, a top anvil plate 340 is pivotally coupled to the proximal end 305 of the channel member 302 by a pair of trunnion pins 342 that are sized to be received in oval-shaped pivot holes 311 provided through the side walls 309 of the channel member 302. In various embodiments, the top anvil plate 350 is fabricated from rigid material to minimize any deflection or warpage of the top anvil member during use. The top anvil plate 340 is designed to mate with a bottom anvil plate 350 that is attached to the proximal end 504 of the staple cartridge 500 by at least one, and preferably two springs 352. In various embodiments, one portion 353 of each of the springs 352 is attached to the cartridge body 502 by adhesive, slots, mechanical fasteners, etc. The other portion 354 of each of the springs 352 is attached to the bottom surface 356 of the bottom anvil plate 350 by adhesive, slots, mechanical fasteners, etc. See Figure 2. As can be seen in Figure 8, the bottom surface 356 of the bottom anvil plate 350 has a series of staple forming pockets 358 formed therein. The reader will understand that in various embodiments, the bottom anvil plate 350 may be attached to the cartridge body 502 such that the staple forming pockets 358 register with corresponding staple-receiving pockets 512 in the cartridge body 502. It will be understood that the staple forming pockets 358 serve to close the staples 534 as the ends of the staple legs 536 are forced into contact therewith. See Figure 6. In addition, in various embodiments, similar staple pockets (not shown) may be formed in the bottom surface 341 of the top anvil plate 340. As the present Detailed Description continues, the reader will appreciate that such top anvil plate construction enables the end effector 300 to be effectively used with cartridges 500 that do not have bottom anvils associated therewith as well as cartridges 500 that have such bottom anvils attached or otherwise associated therewith.

In various embodiments, the bottom anvil plate 350 maybe directly attached to the cartridge body 302 and packaged therewith. However, in other embodiments, the bottom anvil plate 350 may be separately packaged and installed on the cartridge 500 prior to use by the user. For example, the top surface 503 of the cartridge body 502 may be provided with a pair of slots or other retention formations that are adapted to retain the portions 353 of the springs 352 therein. In other embodiments for example, the bottom anvil plate 350 may be attached to the top anvil plate by snapping, etc. Thus, it will be appreciated that in various embodiments of the present invention, the bottom anvil plate 350 does not necessarily have to be attached to the staple cartridge body 502. The bottom anvil plate 350 merely has to be "associated with" a particular corresponding staple cartridge. As used in the context, the term "associated with" means that the bottom anvil 350 plate is configured to be aligned with a particular staple cartridge such that when the staples in the cartridge are deployed into the bottom anvil plate 350, the bottom anvil plate 350 causes the staples 534 to be formed in a desired manner and is intended to encompass those arrangements wherein the bottom anvil plate 350 is directly coupled to the cartridge body 502, temporarily attached to the top anvil plate 340, and those arrangements wherein the bottom anvil plate 350 is otherwise held in registry with the staple cartridge body 502 between the top anvil plate 340 and the staple cartridge body 502 during the deployment of the staples 534 into tissue clamped in the end effector 300.

In various embodiments for example, the bottom anvil plate 350 is provided with a pair of stiffener rails 360 that extend along each lateral edge 359 of the bottom anvil plate 350 such that when the cartridge/bottom anvil plate assembly, generally designated as 370, is installed in the elongate channel 302, the top anvil plate 340 is received between the stiffener rails 360 to form an anvil assembly 372. The reader will appreciate that the stiffener rails 360 serve to stiffen the anvil assembly 372 and may serve to snappingly attach the bottom anvil plate 350 to the top anvil plate 340. In various embodiments, the bottom anvil plate 350 may be stamped or otherwise folded or formed out of sheet metal or similar material or thin deflectable plate material, such that after use, it is disposed of with the staple cartridge body 502. Hence, for various embodiments of the present invention, the bottom anvil plate 350 may be referred to herein as a "disposable anvil plate" or a "disposable anvil".

In addition, a longitudinal slot 362 may be provided through the center of the bottom anvil plate 350 for receiving the upper end of the knife assembly 320 therethrough. A sufficient amount of space may be provided between the bottom surface 341 of the top anvil plate 340 and the upper surface 357 of the bottom anvil plate 350 such that the laterally extending guide tabs 330 formed on the upper end of the knife assembly 320 serve to ride on the upper surface 357 of the bottom anvil plate 357 and urge the bottom anvil plate 350 toward the cartridge body 502 as the knife assembly 320 and wedge sled 326 are driven through the cartridge 520 to cut the tissue and deploy the staples 534. In addition, a longitudinal slot 343 may be provided in the bottom surface 341 of the top anvil plate 340 to accommodate the top end of the knife assembly 320 therein.

Another unique and novel aspect of the present invention is the ability of various end effector embodiments to be effectively used in connection with staple cartridges that have different staple line lengths "L". For example, various embodiments of the present invention employ tissue stops 364 on the bottom anvil plate 350. In addition, to support these "first" tissue stops 364 on the anvil plate 350, a pair of top tissue stops 344 are formed on the top anvil plate 340 to backup and abut (otherwise "cooperate with") the first tissue stops 364 when the cartridge assembly 500 is installed in the channel as shown in Figures 8-11. The skilled artisan will understand that the location of the first tissue stops 364 on the bottom anvil plate 350 may be specifically tailored to the positions of the innermost staple-receiving pockets 512 on the disposable staple cartridge 500 to which it is attached. As will be discussed in further detail below, this unique and novel aspect of the present invention enables the use of a variety of different cartridges in the end effector that have differing lengths "L" of staple lines (for example 45mm, 60mm, etc).

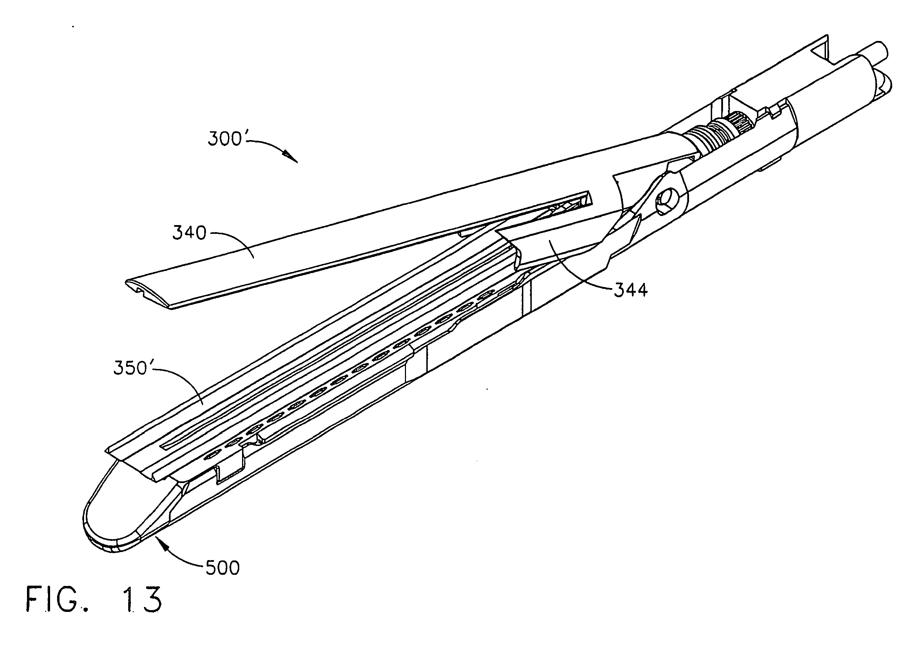

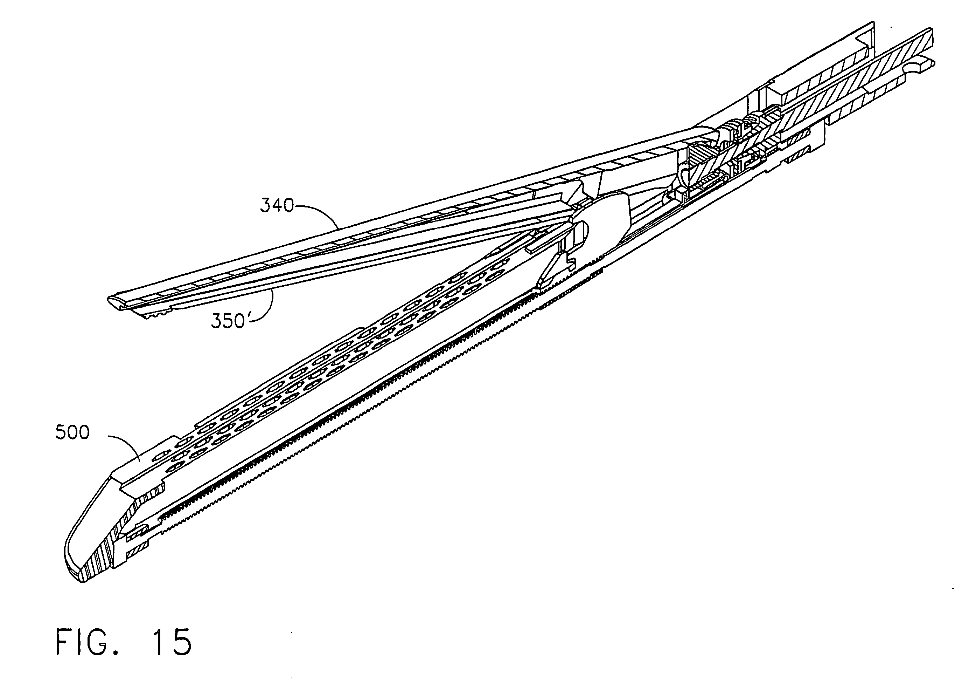

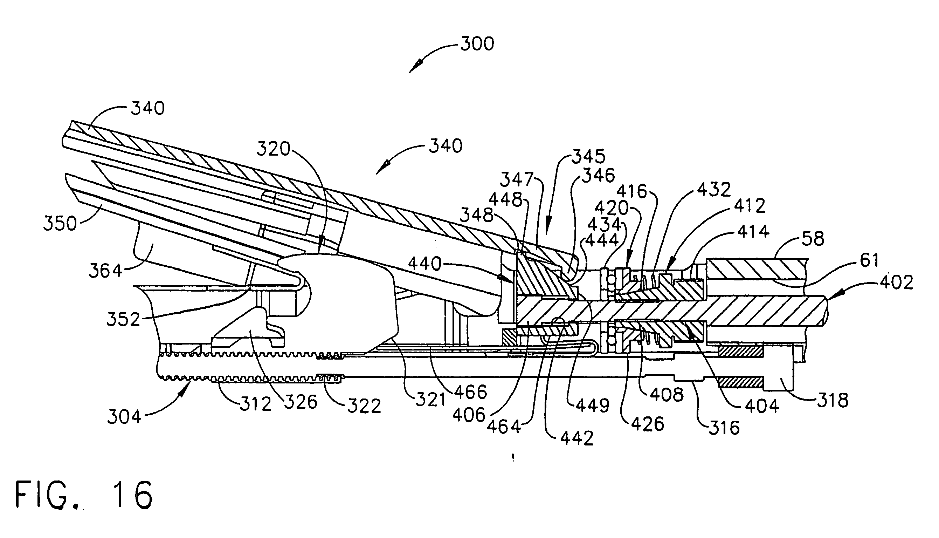

In other embodiments wherein no staple forming pockets are formed in the bottom surface 341 of the top anvil plate 340, a bottom anvil plate 350 may be employed as described above for those staple cartridges 500 that have longer lines of staples that can rely solely on the top tissue stops 344 to orient the tissue so as to prevent the tissue from going all the way back into the anvil/cartridge arrangement and thereby result in tissue being cut but not stapled. Thus, as can be seen in Figures 15-19. To remove the spent cartridge 500 from the channel 302, the bottom anvil plate 350 is depressed and the cartridge and bottom anvil plate 350 can be withdrawn out the distal end 303 of the elongate channel 302. See Figure 13.

A drive assembly for operating various embodiments of the end effector 300 will now be described with reference to Figures 2 and 18-29. As can be seen in Figures 2, and 17-19, a distal drive shaft portion 402 extends through a drive shaft hole 61 in the distal spine tube 58. The distal drive shaft portion 402 may extend directly to a drive motor arrangement in the control handle 6 or it may be articulated to enable the end effector 300 to be pivoted relative to the shaft or closure tube assembly that connects the end effector 300 to the control handle 6. Although such aspects are not the primary focus of this disclosure, various alternative arrangements will be briefly discussed below.

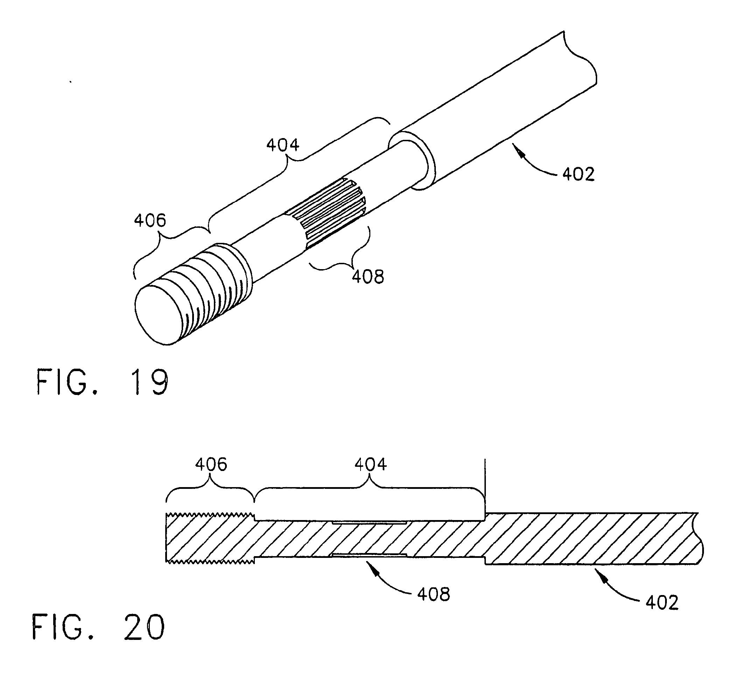

As can be seen in Figures 20, 21, 28, and 29, the distal drive shaft portion 402 has a clutch-receiving portion 404 and a closure thread 406 formed thereon. A clutch assembly 410 is slidably received on the clutch-receiving portion 404 of the drive shaft portion 402. As can be seen in Figures 2, 22 and 23, the clutch assembly 410 includes a collet-like tapered clutch member 412 that has a drive gear 414 integrally formed on its proximal end 413. The drive gear 414 meshes with a transfer gear 450 that in turn meshes with the knife screw gear 316. See Figures 2, 6 and 7. Thus, when the clutch assembly 410 drivingly engages the distal drive shaft portion 402, the drive gear 414 rotates the transfer gear 450 which, in turn rotates the knife screw gear 316.

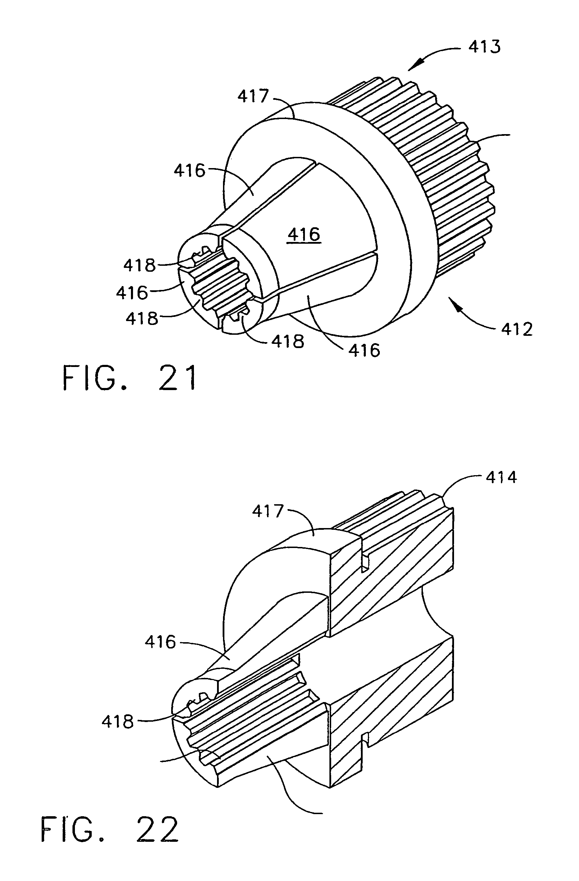

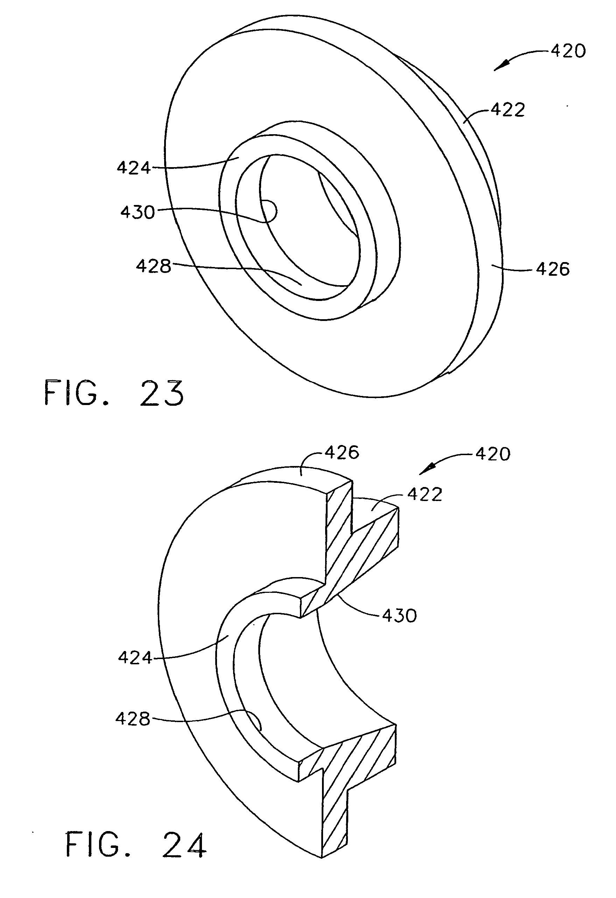

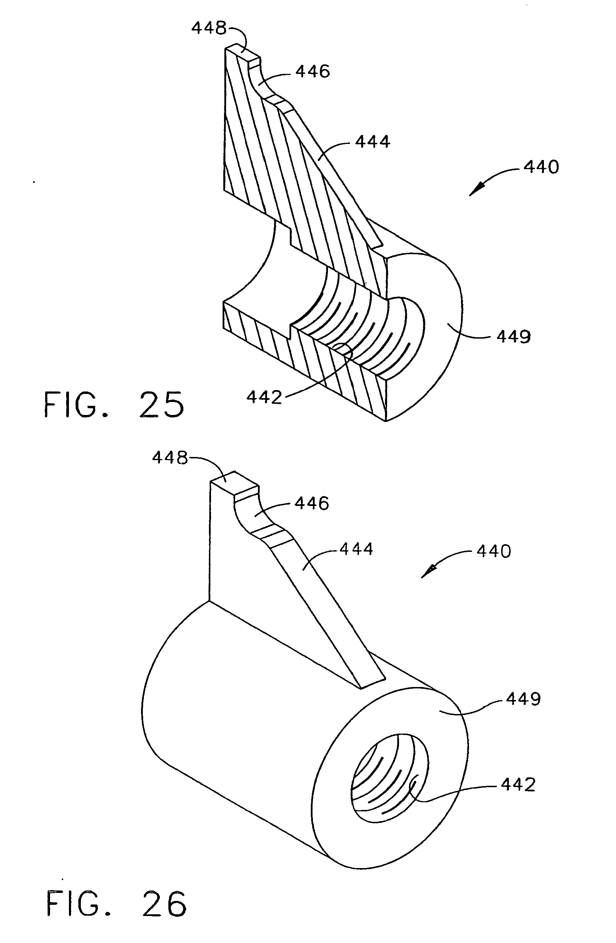

A series of four tapered sections 416 are formed on the distal end 415 of the tapered clutch member 412. A series of male splines 418 are formed in the interior of the tapered sections 416. See Figures 22 and 23. The male splines 418 are adapted to selectively engage a female spline section 408 formed on the distal drive shaft portion 402 as will be discussed in further detail below. See Figures 20 and 21. The clutch assembly 410 further includes a clutch plate 420 that is received on the tapered sections 416 of the tapered clutch member 412. As can be seen in Figures 24 and 25, the clutch plate 420 has a proximal hub portion 422 and a distal hub portion 424 that is separated by a flange portion 426. A cylindrical distal hole portion 428 extends through the distal hub portion 424 and a tapered proximal hole 430 extends through the flange portion 426 and the proximal hub portion 422. See Figure 25. The hole portions 428, 430 enable the clutch plate 420 to be slidably received on the drive shaft 402 and slide onto the tapered clutch member 412. A clutch opening spring is provided between a flange portion 417 formed on the tapered clutch member 412 and the flange portion 426 of the clutch plate 420 and a thrust bearing 434 is also journaled on the clutch-receiving portion 404 adjacent to the clutch plate 420. See Figures 2, 17 and 19.

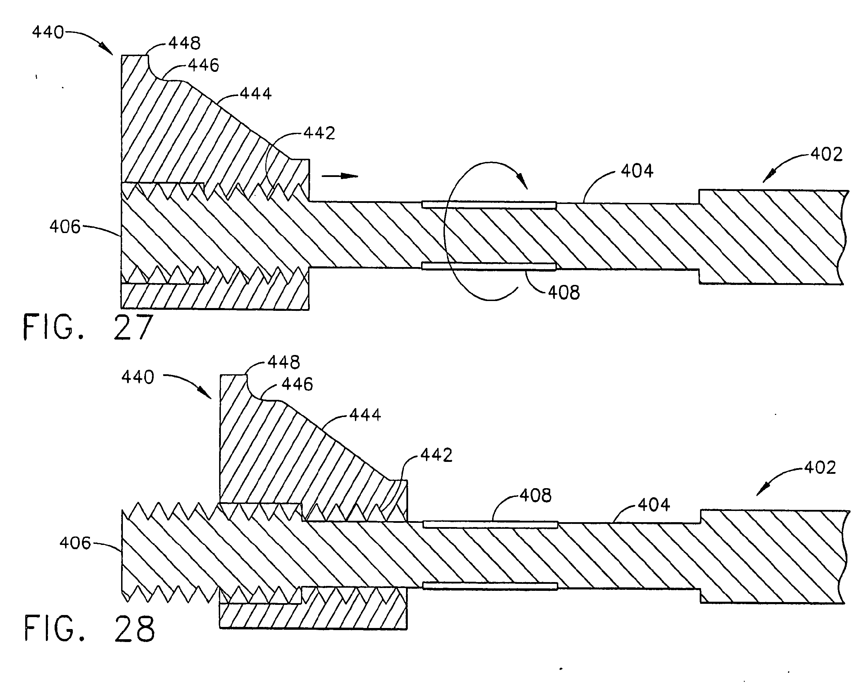

Also in various embodiments, a closure nut 440 is received on the distal drive shaft portion 402. As can be seen in Figures27-29, the closure nut 440 has a threaded hole portion 442 extending partially therethrough to enable it to be threaded onto the closure thread 406 on the distal drive shaft portion 402. As can be further seen in those Figures, the closure nut 440 has an upstanding closure ramp 444 protruding therefrom. The top of the closure ramp 444 terminates in a radiused portion 446 that extends to an upstanding closure tab 448 that is adapted to engage a downwardly protruding closure hook 346 formed on the proximal end 345 of the top anvil plate 340.

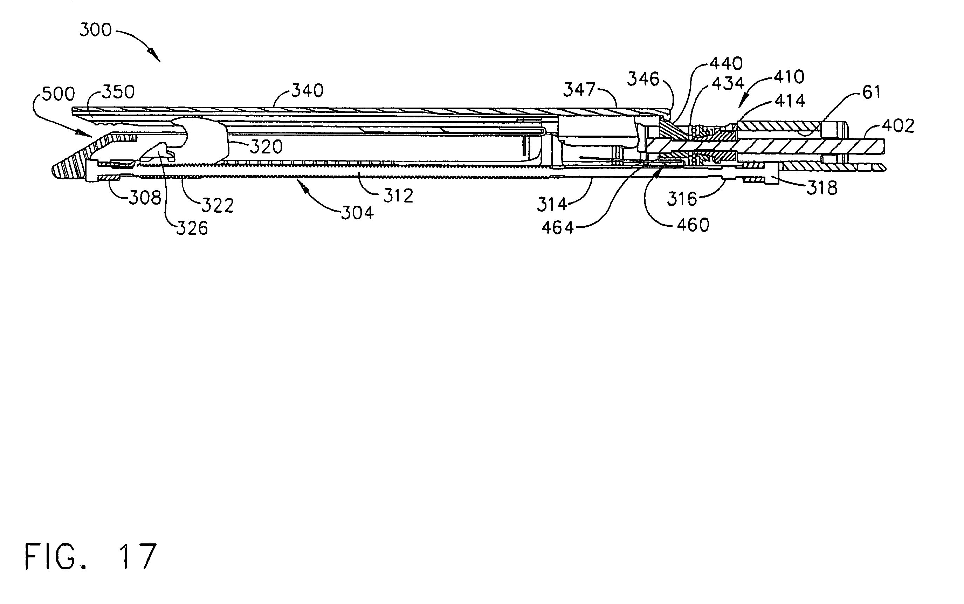

More specifically and with reference to Figures 17 and 19, the proximal end 345 of the top anvil plate 340 has an anvil closure arm portion 347 protruding proximally therefrom that terminates in a downwardly extending closure hook 346. As can also be seen in those Figures, the bottom surface of the anvil closure arm 347 has a tab relief groove 348 therein for receiving the closure tab 348 when the closure nut 440 is advanced to its most distal position (shown in Figures 17 and 28). Also in various embodiments, a closure lock spring 460 is attached to the bottom of the channel member 302, by mechanical fastener arrangements or adhesive. The closure lock spring 460 has an upper portion 462 that terminates in an upstanding retainer lip 464. In addition, longitudinally extending retainer arm is rigidly attached to the upper portion 462 of the closure lock spring 460.

The operation of various embodiments of the present invention will now be described with reference to Figures 9, 11, 17-19 and 28-29. Figures 9, 11, and 17 illustrate the end effector 300 in an open position. As can be seen in those Figures, when in the open position, the closure hook arm 346 of the top anvil plate 340 is riding on the ramp portion 444 of the closure nut 440. When in this position, the top anvil plate 340 is pivoted to the open position. Also when in this position, the end of the retainer arm 466 that is attached to the closure lock spring 460 is in contact with a ramp surface 321 formed on the proximal end of the knife assembly 320. As the knife assembly 320 moves proximally, the end of the retainer arm 466 contacts the ramp surface 321 on the proximal end of the knife assembly 320 and serves to cause the retainer arm 466 to bias the upper portion 462 of the closure lock spring 460 downward toward the bottom of the elongate channel 302. When the knife assembly 320 moves distally away from the retainer arm 466, the upper portion 462 of the closure lock spring 460 is permitted to spring upward to enable the retainer lip 464 to engage the closure nut 440 as will be further discussed below. As can also be seen in Figures, when in the open position, the closure tab 448 is located within the tab relief groove 348 in the bottom surface of the top anvil plate 340.

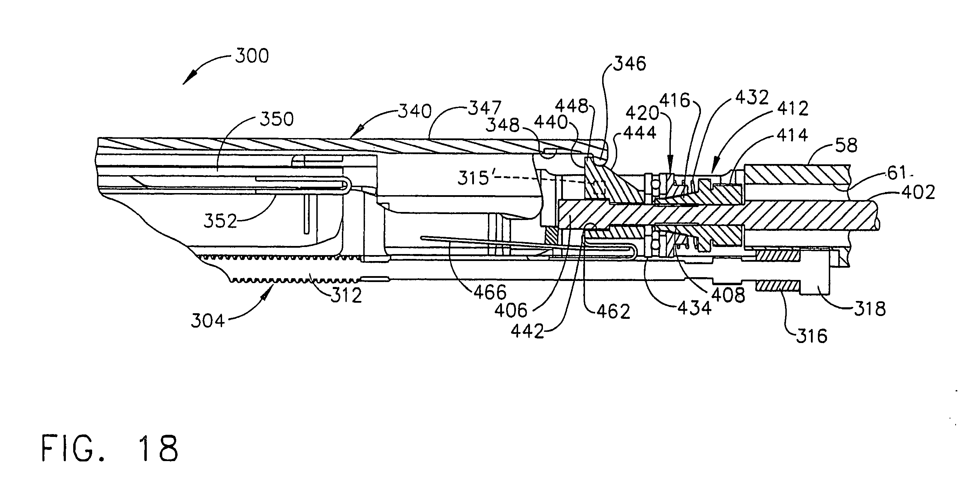

The reader will appreciate that when the end effector 300 is in the open position depicted in Figures 9, 11 and 17, the user can install a disposable cartridge assembly 500 in the elongate member 302. When the distal drive shaft portion 402 is rotated in a first direction, the closure thread 406 thereon threadably drives the closure nut 440 in the proximal direction (direction "A" in Figure 28) until the closure threads 406 disengage the threaded hole 442 in the closure nut 440. See Figure 29. As the closure nut 440 is driven proximally, the closure hook 346 on the anvil closure arm 347 rides up the ramp 444 of the closure nut 440 until it rides into the radiused portion 446 and contacts the closure tab 448. Such movement of the closure nut 440 serves to "pull" the anvil assembly 372 (the top anvil plate 240 and the bottom anvil plate 350) to the closed position as shown in Figures 18 and 19.

As the closure nut 440 is driven in the proximal direction, the proximal end 449 of the closure nut 440 contacts the thrust bearing 434 which forces the clutch plate 420 in the proximal direction against the force of clutch opening spring 432. Further travel of the closure nut 440 in the proximal direction drives the clutch plate 420 onto the tapered sections 416 of the tapered clutch member 412 which causes the male splines 418 therein to engage the female splines 408 on the distal drive shaft portion 402. Such engagement of the male splines 418 in the tapered clutch member 412 with the female splines on the distal drive shaft portion 402 causes the tapered clutch member 412 and the drive gear 414 to rotate with the distal drive shaft portion 402. Drive gear 414, in turn, rotates the knife screw gear 316 which causes the knife screw to rotate and drive the knife assembly distally ("B: direction").

As the knife assembly 320 is driven distally, the laterally extending guide tabs 330 engage the top surface of the bottom anvil plate 350 and serve to pull the bottom anvil plate 350 downward to further clamp the tissue between the bottom anvil plate 350 and the cartridge body 502. The knife blade 324 on the knife assembly 320 cuts the tissue and the cams 328 on the wedge sled 326 serves to drive the staple supporting drivers 532 upward which drive the staples 534 toward the bottom anvil plate 350. As the legs 536 of the staples 534 are driven into the corresponding staple forming pockets 358 in the bottom anvil plate 350, they are folded over. See Figure 6. The tissue stops 364 cooperate with the top tissue stops 344 on the top anvil plate 340 to prevent the tissue from passing too far into the cartridge/anvil arrangement beyond the innermost staples and thus serves to prevent tissue from being cut that would not be stapled.

When the knife assembly 320 moves distally, the retainer arm 466 no longer is in contact with the ramp surface 321 of the knife assembly 320 which enables the retainer arm 466 and the upper portion 462 of the closure lock spring 460 to spring upwardly which further enables the retainer lip 464 on the closure lock spring 460 to retainingly engage the distal end of the closure nut 440 to prevent it from moving distally. See Figures 17 and 18. By virtue of its contact with the closure nut 440 which is in contact with the thrust bearing 434, the retainer lip 464 serves to retain the clutch assembly 410 engaged with the distal drive shaft portion 402 until the knife assembly 320 once again returns to contact the retainer arm 464. After the knife assembly 320 has been driven to its final distal position as shown in Figure 17, it activates a conventional sensor or contact 313 mounted within the elongate channel 302 and signals the control motor to stop driving the drive shaft 402. See Figure 2. Those of ordinary skill in the art will understand that a variety of different control arrangements could be employed to control the drive shaft 402. For example, when the knife assembly 310 reaches its distal-most position and activates the sensor 313, the control system 610 housed within the handle 6 could automatically reverse the drive motor 600 therein and cause the drive shaft portion 402 and knife screw to reverse direction (e.g., move in the proximal "A" direction). In various other embodiments, the control system 610 may simply stop the drive motor 600 and then require the surgeon to activate a button 30 to cause the motor 600 to reverse. In still other arrangements, the control system 610 may institute a predetermined timed delay between the time that the reversing sensor 313 is activated and the time that the motor 600 is reversed.

As the knife assembly 320 moves in the proximal direction on the knife screw 304, the closure threads 406 on the drive shaft 402 begin to screw back into the threaded hole portion 442 in the closure nut 440. During this process, the ramp surface 321 of the knife assembly 320 again contacts the end of the retainer arm 466 which serves to bias the upper portion 462 of the closure lock spring 460 toward the bottom of the elongate channel 302 to permit the retainer lip 464 to disengage from the distal end of the closure nut 440 thereby permitting the clutch opening spring 432 to bias the clutch assembly 410 and closure nut 440 distally. As the closure nut 440 moves distally, the closure hook 346 on the top anvil plate rides up the ramp 444 on the closure nut until the closure nut 440 reaches the open position wherein the closure tab 448 contacts the tab relief groove 348 in the top anvil plate 340 and the closure nut 440 moves the anvil assembly 372 to the open position. A second conventional sensor or contact 315 is mounted within the proximal end portion 305 of the elongate channel 302 for sensing when the closure nut 440 is in the open position and communicates with the motor to cause it to stop. See Figure 2.

As indicated above, a variety of different motor/control arrangements may be employed to power the drive shaft portion 402. For example, in various embodiments when the closure trigger 18 is actuated, that is, drawn in by a user of the instrument 10, the motor 600 may commence the above described closing process. A third sensor 315' may be used in the elongate channel member 302 to sense when the closure nut 404 has moved into the closed position (shown in Figure). When the third sensor 315' senses that the closure nut 440 is in that position, the sensor 315' may cause the motor 600 to stop rotating. Thereafter, if the surgeon is satisfied with the clamping of the tissue in the end effector 300, the surgeon may actuate the firing trigger 20 or other actuator arrangement to activate the motor 600 to rotate the drive shaft 402 which drives the knife screw 304 in the above-mentioned manner.

It will be appreciated from the foregoing discussion, that various embodiments of the present invention represent vast improvements over prior surgical cutting and fastening end effectors and cartridges to be used with such instruments. In particular, various embodiments of the present invention enable different cartridge configurations to be used in the same end effector. Other features of at least some of the embodiments of the present invention involve the provision of staple cartridges that are more economical to manufacture and which require lower firing forces to be generated by the instrument to cut and staple tissue. In addition, another feature of various embodiments of the present invention provide end effectors that can be used to repeatedly deploy staples into thick tissue utilizing firing forces that are generally lower than those firing forces required by at least some prior end effector arrangements.

Any patent, publication, or information, in whole or in part, that is said to be incorporated by reference herein is incorporated herein only to the extent that the incorporated material does not conflict with existing definitions, statements, or other disclosure material set forth in this document. As such the disclosure as explicitly set forth herein supersedes any conflicting material incorporated herein by reference.

The invention which is intended to be protected is not to be construed as limited to the particular embodiments disclosed. The embodiments are therefore to be regarded as illustrative rather than restrictive.

## Claims

1. A disposable staple cartridge (500) for a reusable surgical cutting and fastening instrument (10), said staple cartridge, comprising:
a disposable cartridge body (502) operably supporting a plurality of staples therein, such that said staples are adapted to be sequentially fired therefrom by an axially movable driving portion of the surgical cutting and fastening instrument;
a disposable anvil supported relative to said cartridge body such that said disposable anvil is movable between a closed position wherein a bottom surface (350) of said disposable anvil is adjacent a top surface (503) of said cartridge body and an open position wherein the bottom surface of said disposable anvil is spaced away from said top surface of said cartridge body to permit tissue to be inserted therebetween; and
at least one tissue stop (364) on said disposable anvil for contacting the tissue inserted between the bottom surface of the disposable anvil and the top surface of said cartridge body to orient the tissue relative to the staples supported within said cartridge body;
**characterised by** said disposable anvil having a stiffener rail (360) extending along each lateral edge of its top surface to releasably engage a top anvil plate (340) on the surgical cutting and fastening instrument.

2. The disposable staple cartridge of claim 1 wherein said disposable anvil is fabricated from thin deflectable plate material.

3. The disposable staple cartridge of claim 1 wherein a plurality of staple forming pockets (512) corresponding to said plurality of staples in said cartridge body are formed in said bottom surface of said disposable anvil.

4. The disposable staple cartridge of claim 1 wherein a proximal end of said disposable anvil is attached to a proximal end (504) of said cartridge body by at least one spring.

5. The disposable staple cartridge of claim 1 wherein said at least one tissue stop comprises a first tissue stop protruding downwardly from one lateral side of said disposable anvil and a second tissue stop protruding downwardly from a second lateral side of said disposable anvil.

6. The disposable staple cartridge of claim 1 wherein said disposable anvil has a longitudinally extending slot portion (362) therein to enable a portion of a knife assembly (320) of the surgical cutting and fastening instrument to protrude therethrough such that lateral extending guide tabs (330) on the knife assembly serve to deflect the disposable anvil toward said top surface of said cartridge body as the knife assembly is driven through said cartridge body and said longitudinally extending slot in said disposable anvil.

7. The disposable staple cartridge of claim 1 wherein said disposable anvil is fabricated from sheet metal material.

8. The disposable staple cartridge of claim 1 further comprising a plurality of staple drivers operably supposed in said cartridge body, each said staple driver supporting at least one staple thereon and being movably supporter within said cartridge body such that when contacted by a driven wedge cam (326) of the surgical cutting and fastening instrument, each said staple driver drives the staples supported thereon into forming contact with said disposable anvil.

## Patentansprüche

1. Wegwerfklammerkassette (500) für ein wiederverwendbares chirurgisches Schneid- und Befestigungsinstrument (10), umfassend:
einen Wegwerfkassettenkörper (502), der mehrere Klammern derart betriebsbereit darin lagert, dass die Klammern angepasst sind, der Reihe nach mittels eines axial bewegbaren Antriebsabschnitts des chirurgischen Schneid- und Befestigungsinstruments geschossen zu werden;
einen Wegwerfamboss, der bezüglich des Kassettenkörpers derart gelagert ist, dass der Wegwerfamboss zwischen einer geschlossenen Position, in der eine untere Fläche (350) des Wegwerfamboss benachbart zu einer oberen Fläche (503) des Kassettenkörpers ist,
und einer offenen Position beweglich ist, in der die untere Fläche des Wegwerfambosses in einem Abstand zu der oberen Fläche des Kassettenkörpers angeordnet ist, um das Einbringen von Gewebe dazwischen zu ermöglichen; und
wenigstens einen Gewebeanschlag (364) an dem Wegwerfamboss zum Kontaktieren des zwischen der unteren Fläche des Wegwerfamboss und der oberen Fläche des Kassettenkörpers eingebrachten Gewebes, um das Gewebe bezüglich der innerhalb des Kassettenkörpers gelagerten Klammern auszurichten;
**dadurch gekennzeichnet, dass** der Wegwerfamboss eine Versteifungsschiene (360) aufweist, die sich längs jedes lateralen Rands dessen oberer Fläche erstreckt, um mit einer oberen Ambossplatte (340) an dem chirurgischen Schneid- und Befestigungsinstrument lösbar in Eingriff zu gelangen.

2. Wegwerfklammerkassette nach Anspruch 1, wobei der Wegwerfamboss aus dünnem, biegbarem Plattenmaterial gefertigt ist.

3. Wegwerfklammerkassette nach Anspruch 1, wobei mehrere Klammerformtaschen (512) in der unteren Fläche des Wegwerfamboss ausgebildet sind, die mit den mehreren Klammern in dem Kassettenkörper korrespondieren.

4. Wegwerfklammerkassette nach Anspruch 1, wobei ein proximales Ende des Wegwerfamboss an einem proximalen Ende (504) des Kassettenkörpers mit wenigstens einer Feder angebracht ist.

5. Wegwerfklammerkassette nach Anspruch 1, wobei der wenigstens eine Gewebeanschlag einen ersten Gewebeanschlag, der von einer lateralen Seite des Wegwerfambosses abwärts hervorsteht, und einen zweiten Gewebeanschlag umfasst, der von einer zweiten lateralen Seite des Wegwerfamboss abwärts hervorsteht.

6. Wegwerfklammerkassette nach Anspruch 1, wobei der Wegwerfamboss in sich einen sich längs erstreckenden Schlitzabschnitt (362) aufweist, um einem Abschnitt einer Schneideanordnung (320) des chirurgischen Schneid- und Befestigungsinstruments zu ermöglichen, dort hindurch derart vorzustehen, dass auf der Schneideanordnung sich lateral erstreckende Führungsstreben (330) dazu dienen, den Wegwerfamboss zu der oberen Fläche des Kassettenkörpers hin zu biegen, wenn die Schneideanordnung durch den Kassettenkörper und den sich in dem Wegwerfamboss längs erstreckenden Schlitz hindurch angetrieben ist.

7. Wegwerfklammerkassette nach Anspruch 1, wobei der Wegwerfamboss aus einem Blechmaterial gefertigt ist.

8. Wegwerflclammerkassette nach Anspruch 1, umfassend mehrere Klammerantriebe, die in der Klammerkassette betriebsbereit gelagert sind, wobei jeder Klammerantrieb wenigstens eine Klammer auf sich lagert und innerhalb des Kassettenkörpers derart beweglich gelagert ist, dass, wenn er von einer angetriebenen Keilnocke (326) des chirurgischen Schneid- und Befestigungsinstruments kontaktiert ist, jeder Klammerantrieb die darauf gelagerten Klammern in formenden Kontakt mit dem Wegwerfamboss antreibt.

## Revendications

1. Cartouche d'agrafes jetable (500) pour un instrument de coupe et de fixation chirurgical réutilisable (10), ladite cartouche d'agrafes comprenant :
un corps de cartouche jetable (502) supportant de manière opérationnelle une pluralité d'agrafes à l'intérieur de ce dernier, de sorte que lesdites agrafes sont adaptées pour être séquentiellement déclenchées de ce dernier par une partie d'entraînement axialement mobile de l'instrument de coupe et de fixation chirurgical ;
une enclume jetable supportée par rapport audit corps de cartouche de sorte que ladite enclume jetable est mobile entre une position fermée dans laquelle une surface inférieure (350) de ladite enclume jetable est adjacente à une surface supérieure (503) dudit corps de cartouche et une position ouverte dans laquelle la surface inférieure de ladite enclume jetable est espacée de ladite surface supérieure dudit corps de cartouche pour permettre d'insérer le tissu entre elles ; et
au moins une butée de tissu (364) sur ladite enclume jetable pour entrer en contact avec le tissu inséré entre la surface inférieure de ladite enclume jetable et la surface supérieure dudit corps de cartouche afin d'orienter le tissu par rapport aux agrafes supportées dans ledit corps de cartouche ;
**caractérisée par** ladite enclume jetable ayant un rail de renfort (360) s'étendant le long de chaque bord latéral de sa surface supérieure pour mettre en prise de manière amovible une plaque d'enclume supérieure (340) sur l'instrument de coupe et de fixation chirurgical.

2. Cartouche d'agrafes jetable selon la revendication 1, dans laquelle ladite enclume jetable est fabriquée à partir d'un matériau de plaque fin déviable.

3. Cartouche d'agrafes jetable selon la revendication 1, dans laquelle on forme une pluralité de poches de formation d'agrafe (512) correspondant à ladite pluralité d'agrafes dans ledit corps de cartouche dans ladite surface inférieure de ladite enclume jetable.

4. Cartouche d'agrafes jetable selon la revendication 1, dans laquelle une extrémité proximale de ladite enclume jetable est fixée sur une extrémité proximale (504) dudit corps de cartouche par au moins un ressort.

5. Cartouche d'agrafes jetable selon la revendication 1, dans laquelle ladite au moins une butée de tissu comprend une première butée de tissu faisant saillie vers le bas à partir d'un côté latéral de ladite enclume jetable et une seconde butée de tissu faisant saillie vers le bas à partir d'un second côté latéral de ladite enclume jetable.

6. Cartouche d'agrafes jetable selon la revendication 1, dans laquelle ladite enclume jetable a une partie de fente (362) s'étendant longitudinalement à l'intérieur de celle-ci pour permettre à une partie d'un ensemble de lame (320) de l'instrument de coupe et de fixation chirurgical de faire saillie à travers cette dernière de sorte que les languettes de guidage (330) s'étendant latéralement sur l'ensemble de lame servent à dévier l'enclume jetable vers ladite surface supérieure dudit corps de cartouche lorsque l'ensemble de lame est entraîné à travers ledit corps de cartouche et ladite fente s'étendant longitudinalement dans ladite enclume jetable.

7. Cartouche d'agrafes jetable selon la revendication 1, dans laquelle ladite enclume jetable est fabriquée à partir d'un matériau en tôle.

8. Cartouche d'agrafes jetable selon la revendication 1, comprenant en outre une pluralité de dispositifs d'entraînement d'agrafe supportée dans ledit corps de cartouche, chaque dispositif d'entraînement d'agrafe supportant au moins une agrafe sur ce dernier et étant supporté de manière mobile à l'intérieur dudit corps de cartouche de sorte que lorsqu'il est en contact avec une came de cale entraînée (326) de l'instrument de coupe et de fixation chirurgical, ledit dispositif d'entraînement d'agrafe entraîne les agrafes supportées sur celui-ci en contact de formation avec ladite enclume jetable.
